# FASCICULE DE BREVET EUROPEEN

(11) **EP 1 649 807 B1**
(45) Date de publication et mention de la délivrance du brevet: **16.01.2008**
(21) Numéro de dépôt: 05300837.1
(22) Date de dépôt: 18.10.2005
(51) Int. Cl.: A61B 5/103, A45D 42/10, F21V 9/14, G02B 27/28

(54) **Dispositif permettant l'observation du visage**
Vorrichtung für die Beobachtung des Gesichtes
Device allowing the observation of the face

(30) Priorité: 22.10.2004 FR 0452419
(43) Date de publication de la demande: 26.04.2006
(73) Titulaire: L'ORÉAL, 75008 Paris (FR)
(72) Inventeur: Bazin, Roland, 91570, Bievres (FR)
(74) Mandataire: Tanty, François

(56) Documents cités:
- EP-A- 1 433 418
- US-A- 4 070 096

## Description

La présente invention concerne notamment un dispositif permettant l'observation du visage.

La demande EP-A1-1 433 418 concerne un procédé et un appareil pour identifier des imperfections sur la peau d'une personne. Le dispositif comporte une caméra et deux sources lumineuses, l'une de longueur d'onde visible et l'autre dans l'ultraviolet pour générer différentes images de la peau.

On connaît par le brevet US 3 794 828 un dispositif comportant un miroir et permettant d'éclairer le visage avec différentes conditions d'illumination.

Les brevets US 3 711 182 et US 4 070 096 divulguent des dispositifs comportant un miroir et un système d'éclairage en lumière polarisée permettant d'éliminer la brillance

La brillance de la peau est susceptible d'évoluer au cours de la journée. En particulier, la peau peut être relativement mate le matin et sa brillance peut augmenter au cours du temps, par exemple en raison de la sécrétion de sébum ou de sueur.

Certains produits, par exemple les fonds de teint, peuvent permettre d'atténuer cette brillance.

Cependant, le produit appliqué ne doit pas complètement éliminer la brillance, car une certaine brillance résiduelle reste souhaitable pour conférer de l'éclat au teint. Le niveau de brillance à conserver dépend non seulement de la nature et de la teinte de la peau mais également du souhait du consommateur.

La sélection d'un produit permettant d'atténuer la brillance peut ainsi s'avérer difficile et il existe un besoin pour aider le consommateur dans ce choix.

Il existe également un besoin pour faciliter la fabrication de produits, notamment de fonds de teint, en fonction de l'apparence, et notamment du niveau de brillance, souhaitée par le consommateur.

L'invention vise à répondre à tout ou partie de ces besoins.

L'invention a ainsi pour objet, selon l'un de ses aspects, un dispositif comportant:
- un système de visualisation comportant l'un au moins
   - d'un miroir et
   - d'un système vidéo comportant une caméra et un écran permettant de renvoyer vers l'utilisateur placé devant la caméra une image acquise par la caméra,
- un premier système de polarisation pour polariser la lumière émise par une source lumineuse dans une première direction,
- un deuxième système de polarisation pour polariser la lumière reçue par le miroir ou la caméra dans une deuxième direction,
- des moyens, mécaniques et/ou électroniques, pour faire varier l'écart angulaire entre les première et deuxième directions, l'écart angulaire étant ainsi modifiable,
- un système de repérage de l'écart angulaire permettant de repérer au moins trois écarts angulaires différents, caractérisé en ce que le système de repérage comporte des graduations.

Grâce à l'invention, l'utilisateur peut percevoir l'effet sur son apparence d'une modification de la brillance et repérer l'écart angulaire qui correspond par exemple à l'apparence souhaitée ou à l'apparence abhorrée ou à gamme d'apparences acceptées ou non acceptées.

Le dispositif peut comporter la source lumineuse, laquelle peut être solidaire ou non du système de visualisation. De préférence, la source lumineuse émet une lumière diffuse. La source lumineuse peut comporter au moins un tube fluorescent, une lampe à incandescence ou une diode électroluminescente.

Le dispositif peut avantageusement comporter deux sources lumineuses disposées de part et d'autre du système de visualisation, afin d'obtenir un éclairage plus uniforme.

La source lumineuse peut encore entourer au moins partiellement le système de visualisation.

La ou les sources lumineuses peuvent par exemple être configurées pour fournir un éclairage proche de la lumière du jour où un éclairage différent, par exemple de type lampe à incandescence.

L'un du premier système de polarisation et du deuxième système de polarisation peut être rotatif. Cette rotation peut s'effectuer par incréments ou de manière continue. Un incrément peut correspondre à quelques degrés, par exemple.

Le système de polarisation qui est rotatif peut être configuré pour tourner entre deux positions extrêmes ou pour tourner sur un tour complet.

Le système de visualisation peut comporter un miroir. Ce miroir peut être porté par exemple par un premier élément de structure et la source lumineuse être portée par un deuxième élément de structure, mobile relativement au premier élément de structure. Le deuxième élément de structure est par exemple articulé sur le premier élément de structure. Le premier élément de structure et le deuxième élément de structure peuvent notamment être configurés pour se rabattre l'un sur l'autre, ce qui peut faciliter le transport du dispositif.

Le miroir peut être circulaire ou non, et être ou non entièrement recouvert par le deuxième système de polarisation.

Le deuxième système de polarisation peut être rotatif.

Le miroir peut être non rotatif et le deuxième système de polarisation peut tourner relativement au miroir. En variante, le miroir peut être rotatif et le deuxième système de polarisation tourner avec le miroir.

Le deuxième système de polarisation peut encore être non rotatif et c'est alors par exemple le premier système de polarisation qui est rotatif.

Dans ce cas notamment, le premier système de polarisation est par exemple de forme annulaire.

Le système de visualisation peut ne pas comporter de miroir mais un système vidéo.

Le système de repérage comporte des graduations. Celles-ci peuvent par exemple s'étendre sur un arc de cercle, notamment sur 90° ou moins de 90°. Le système de repérage peut encore comporter un index pouvant coopérer avec les graduations. Cet index est par exemple défini par un organe d'entraînement du deuxième système de polarisation.

Le système de repérage peut encore comporter un capteur de position angulaire.

Le système de repérage peut comporter des moyens permettant de mémoriser une position.

Le dispositif peut comporter des moyens d'affichage d'une information relative à l'écart angulaire entre les première et seconde directions de polarisation.

Le dispositif peut comporter un système de prise de vue afin de prendre une image de l'apparence sélectionnée par l'utilisateur. Ce système de prise de vue peut comporter par exemple un premier système de polarisation sur un flash et un deuxième système de polarisation sur un objectif d'un appareil de prise de vue, par exemple un appareil photographique argentique ou numérique ou une caméra. La source lumineuse du dispositif peut encore procurer la lumière nécessaire à la prise de vue.

L'invention a encore pour objet, indépendamment ou en combinaison avec ce qui précède, un procédé de détermination d'au moins un niveau de brillance du visage d'une personne, sélectionné par celle-ci comportant les étapes suivantes :
- permettre à la personne de visualiser son visage avec un dispositif comportant:
   - un système de visualisation comportant l'un au moins d'un miroir et d'un système vidéo comportant une caméra devant laquelle la personne peut se placer et un écran permettant de renvoyer vers la personne l'image acquise par la caméra,
   - un premier système de polarisation pour polariser la lumière émise par une source lumineuse dans une première direction, afin d'éclairer le visage,
   - un deuxième système de polarisation pour polariser la lumière reçue par le système de visualisation,
   - des moyens pour faire varier l'écart angulaire entre les première et deuxième directions de polarisation et ainsi le niveau de brillance associé,
- permettre à la personne de modifier ou faire modifier l'écart angulaire entre les première et deuxième directions de polarisation, de manière à pouvoir sélectionner au moins une apparence,
- déterminer l'écart angulaire entre les première et deuxième directions de polarisation pour cette apparence sélectionnée au moins, ledit écart étant représentatif du niveau de brillance sélectionné par la personne.

De préférence, l'apparence sélectionnée correspond à l'apparence préférée par cette personne, mais dans des variantes de mise en oeuvre de l'invention, la personne peut sélectionner une apparence abhorrée ou une gamme d'apparences acceptées ou non acceptées.

On peut enregistrer l'écart angulaire ainsi déterminé, afin de pouvoir le comparer à un autre écart angulaire déterminé ultérieurement ou s'en servir pour prescrire un produit ou un traitement ou fabriquer un produit adapté à la personne.

On peut également enregistrer non pas l'écart angulaire lui-même mais une information représentative de celui-ci, par exemple un niveau de brillance ou d'atténuation de celle-ci.

Le procédé peut être mis en oeuvre alors que le visage est maquillé ou non.

On peut délivrer, en fonction de l'écart angulaire sélectionné, une information concernant la peau et/ou les cheveux de la personne, par exemple quantifier la matité ou la brillance de la peau.

On peut délivrer à la personne un produit ou un conseil d'achat d'un produit en fonction de l'écart angulaire déterminé. Le produit est par exemple choisi parmi plusieurs produits existants. Ces produits existants peuvent être respectivement associés à des valeurs ou plages de valeurs par exemple représentatives de différences entre des écarts angulaires sélectionnés et un angle de 45° entre les première et deuxième directions de polarisation, cet angle de 45° correspondant à une absence d'atténuation ou d'augmentation de la brillance.

Le produit peut encore être un produit personnalisé, notamment formulé *in situ*, par exemple sur un point de vente.

L'invention a encore pour objet, indépendamment ou en combinaison avec ce qui précède, un procédé pour mettre en évidence une variation de l'apparence d'une portion du corps humain d'une personne, notamment du visage, comportant :
- permettre à la personne de visualiser cette portion de son corps avec un dispositif comportant :
   - un système de visualisation comportant l'un au moins d'un miroir et d'un système vidéo comportant une caméra devant laquelle la personne peut se placer et un écran permettant de renvoyer vers cette personne une image acquise par la caméra,
   - un premier système de polarisation pour polariser la lumière émise par une source lumineuse dans une première direction,
   - un deuxième système de polarisation pour polariser la lumière reçue par le système de visualisation dans une deuxième direction,
   - des moyens pour faire varier l'écart angulaire entre les première et deuxième directions,
- permettre à la personne de modifier l'écart angulaire entre les directions de polarisation de manière à sélectionner un premier écart angulaire dans un premier intervalle de temps,
- permettre à la personne de sélectionner un deuxième écart angulaire dans un deuxième intervalle de temps, différent du premier, et
- comparer les premier et deuxième écarts angulaires sélectionnés ou toute information représentative de ceux-ci.

On peut appliquer un produit sur la personne entre le premier et le deuxième intervalle de temps, par exemple un fond de teint. On peut alors délivrer une information concernant un effet du produit en fonction de la comparaison, par exemple le pouvoir d'augmentation de la brillance ou matifiant de ce produit.

Le premier intervalle de temps peut être choisi le matin d'une journée donnée et le deuxième intervalle de temps le soir de cette journée, par exemple. Cela peut permettre par exemple de quantifier l'accroissement de la brillance au cours de la journée

L'invention a encore pour objet, indépendamment ou en combinaison avec ce qui précède, un procédé de prescription comportant les étapes suivantes :
- visualiser une portion du corps d'une personne, notamment son visage, avec un dispositif comportant :
   - un système de visualisation comportant l'un au moins d'un miroir et d'un système vidéo comportant une caméra devant laquelle la personne peut se placer et un écran permettant de renvoyer une image acquise par la caméra,
   - un premier système de polarisation pour polariser la lumière émise par une source lumineuse dans une première direction,
   - un deuxième système de polarisation pour polariser la lumière reçue par le système de visualisation dans une deuxième direction, l'orientation de la première direction relativement à la deuxième direction étant modifiable,
- sélectionner une orientation relative,
- prescrire en fonction d'au moins une information liée à l'orientation relative sélectionnée un produit et/ou un traitement cosmétique.

Le produit sélectionné peut notamment agir sur la brillance de la peau. Le produit est par exemple sélectionné parmi plusieurs produits ayant des pouvoirs matifiants différents.

On peut encore déterminer une caractéristique d'application du produit prescrit en fonction de l'orientation sélectionnée, par exemple la quantité de produit à appliquer ou les régions sur lesquelles celui-ci est à appliquer.

L'invention a encore pour objet, indépendamment ou en combinaison avec ce qui précède, un procédé de formulation d'un produit dans lequel le produit est formulé en fonction de l'écart angulaire déterminé plus haut.

Ce procédé peut comporter les étapes suivantes :
- visualiser au moins une portion du corps d'une personne avec un dispositif comportant :
   - un système de visualisation comportant l'un au moins d'un miroir et d'un système vidéo comportant une caméra devant laquelle la personne peut se placer, et un écran permettant de renvoyer vers la personne une image acquise par la caméra,
   - un premier système de polarisation pour polariser la lumière émise par une source lumineuse dans une première direction,
   - un deuxième système de polarisation pour polariser la lumière reçue par le système de visualisation dans une deuxième direction,
   - des moyens pour faire varier l'écart angulaire entre les première et deuxième directions,
- sélectionner un écart angulaire entre les première et deuxième directions de polarisation,
- formuler le produit en fonction au moins d'une information liée à l'écart angulaire sélectionné.

L'écart angulaire sélectionné peu par exemple correspondre à une apparence préférée de la personne, ou en variante à une apparence abhorrée, ou encore à une gamme d'apparences acceptées ou non acceptées par la personne.

On peut par exemple incorporer dans le produit une charge matifiante ou un composé conférant de la brillance, dans une proportion choisie en fonction de l'orientation sélectionnée.

Le produit peut par exemple être formulé de manière à ce qu'après application de celui-ci, l'apparence préférée corresponde à un écart angulaire proche de 45° entre les première et deuxième directions de polarisation.

L'invention a encore pour objet, indépendamment ou en combinaison avec ce qui précède, un procédé pour promouvoir la vente d'un produit, dans lequel on permet à au moins un consommateur de visualiser au moins une portion de son corps, notamment de son visage et/ou de ses cheveux, en utilisant un dispositif tel que défini plus haut, avec au moins deux orientations différentes de l'un des systèmes de polarisation.

L'invention pourra être mieux comprise à la lecture de la description détaillée qui va suivre, d'exemples de réalisation non limitatifs de l'invention, et à l'examen du dessin annexé, sur lequel :
- la figure 1 est une vue schématique, en perspective, d'un dispositif réalisé conformément à un premier exemple de mise en oeuvre de l'invention,
- les figures 2 à 4 sont des vues analogues à la figure 1 de variantes de mise en oeuvre de l'invention,
- les figures 5 et 6 sont des vues de face d'autres exemples de mise en oeuvre de l'invention, et
- la figure 7 est un schéma en blocs illustrant un exemple de procédé selon l'invention.

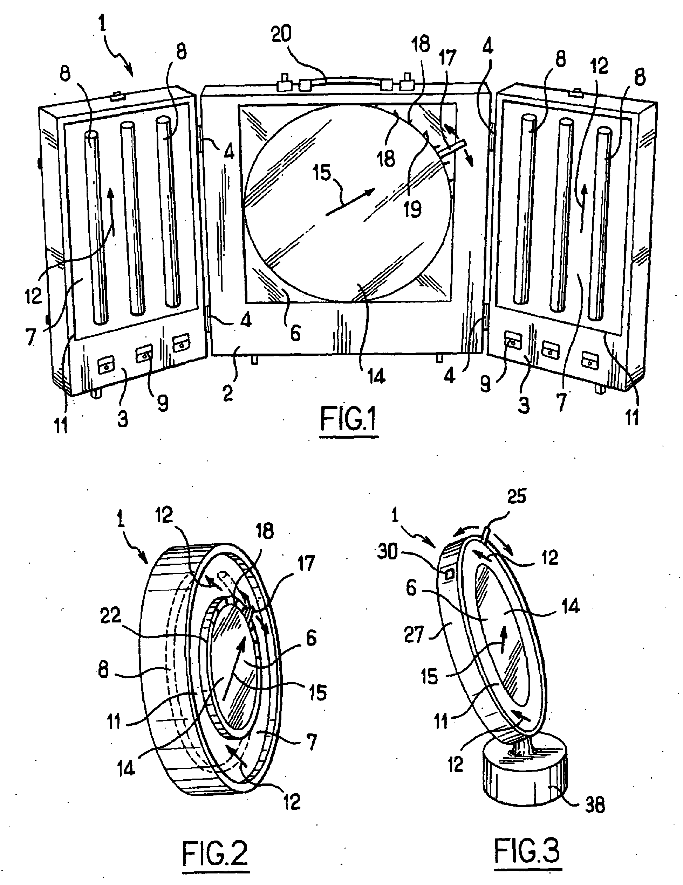

On a représenté à la figure 1 un dispositif 1 réalisé conformément à l'invention, se présentant sous la forme d'une valise, comportant deux éléments de structure. Le premier élément de structure comporte une partie centrale 2 et le deuxième élément de structure deux parties rabattables 3, articulées chacune en 4 sur la partie centrale 2 et formant le couvercle de la valise.

La partie centrale 2 comporte un système de visualisation comportant dans l'exemple illustré un miroir 6, fixe relativement à la partie centrale 2.

Chaque partie rabattable 3 comporte une source lumineuse 7 comprenant plusieurs tubes fluorescents 8 disposés côte à côte de manière à former une source diffuse de lumière. Les tubes 8 peuvent être placés devant un fond blanc ou réfléchissant de la partie rabattable correspondante 3. Dans l'exemple considéré, le dispositif 1 comporte en outre des interrupteurs 9 associés chacun à un tube 8 afin de commander son allumage.

Les parties rabattables 3 peuvent être orientées vers l'avant relativement à la partie centrale 2 lors de l'utilisation, de manière à éclairer au mieux le visage d'une personne placée devant le miroir 6.

De préférence, la puissance de chaque source lumineuse 7 est suffisamment forte pour que l'incidence de l'éclairage par celle-ci du visage de la personne placée devant le miroir 6 soit perceptible.

Un premier système de polarisation 11 est associé à chaque source 7, de manière à polariser la lumière émise par celle-ci dans une direction 12, verticale dans l'exemple considéré.

Le système de polarisation 11 comporte par exemple un film polarisant, lequel est par exemple fixé sur une vitre protégeant les tubes 8.

Un deuxième système de polarisation 14 est associé au miroir 6, pour polariser la lumière selon une deuxième direction 15, laquelle peut présenter une orientation variable relativement à la première direction 12.

Dans l'exemple considéré, le deuxième système de polarisation 14 comporte un disque d'un film polarisant superposé au miroir 6, fixé de manière rotative en son milieu sur la partie centrale 2.

Un organe d'entraînement 17 permet à l'utilisateur de faire tourner le deuxième système de polarisation 14 pour l'amener dans une orientation choisie.

Le montage du deuxième système de polarisation 14 sur la partie centrale 2 peut présenter une friction suffisante pour qu'une fois laissé dans l'orientation choisie le deuxième système de polarisation 14 conserve cette orientation.

Le deuxième système de polarisation peut encore être monté de manière à pouvoir tourner par incréments, par exemple grâce à une roue crantée ou tout autre mécanisme générant des points durs lors du mouvement de rotation.

Un système de repérage est par ailleurs prévu pour déterminer l'orientation dans laquelle le deuxième système de polarisation 14 est laissé.

Ce système de repérage peut par exemple comporter des graduations 18 équi-réparties angulairement et par exemple associées à des valeurs numériques correspondantes, l'organe d'entraînement 17 servant d'index et la lecture de l'angle s'effectuant en cherchant devant quelle graduation 18 l'organe d'entraînement 17 est placé.

Une graduation 19, médiane, peut indiquer l'orientation correspondant à un angle de 45° entre les directions de polarisation 12 et 15.

Bien entendu, l'invention n'est pas limitée à des moyens de repérage particuliers et les graduations 18 peuvent être remplacées par d'autres repères, par exemple par un ou plusieurs caractères alphanumériques, mots, dessins, notamment pictogrammes ou couleurs. Ainsi, le terme « graduation » doit se comprendre avec un sens large.

Les moyens de repérage peuvent par exemple permettre de repérer un niveau de brillance. Par « niveau », il faut comprendre un degré de brillance précis, ou encore une plage de degrés de brillance.

Dans une variante non illustrée, une région de la partie centrale 2, par exemple le miroir 6, peut comporter un index et les graduations ou autres repères peuvent être présents sur le deuxième système de polarisation 14.

Le système de repérage peut encore comporter par exemple au moins un capteur (non représenté) de la position angulaire du deuxième système de polarisation 14.

Le deuxième système de polarisation 14 tourne alors par exemple avec un axe qui entraîne une roue codeuse (non représentée) ou un potentiomètre (non représenté) permettant de transformer un déplacement angulaire en un signal électrique, lequel peut être traité par un circuit électronique approprié et converti par exemple en une valeur numérique renseignant sur l'orientation angulaire, voire en un message plus élaboré, lequel peut être affiché ou non sur un écran ou un afficheur.

Dans l'exemple considéré, c'est la personne qui est placée devant le miroir 6 qui peut elle-même faire tourner le deuxième système de polarisation 14 afin de positionner celui-ci dans l'orientation souhaitée. Dans une variante non illustrée, l'entraînement en rotation du deuxième système de polarisation 14 peut être motorisé, étant commandé par cette personne ou par un opérateur assistant celle-ci.

Dans l'exemple de la figure 1, la valise est munie d'une poignée 20 et peut être transportée si nécessaire assez facilement sur le lieu où l'évaluation doit s'effectuer.

Bien entendu, le dispositif 1 peut ne pas être destiné à être transporté mais être utilisé à poste fixe.

Le dispositif 1 représenté à la figure 2 comporte une source lumineuse 7 annulaire entourant le système de visualisation, lequel comporte comme dans l'exemple de réalisation précédent un miroir 6, dont la forme est toutefois différente puisque circulaire.

La source 7 comporte par exemple un tube fluorescent de forme circulaire entourant le miroir 6 et le premier système de polarisation 11 un film polarisant de forme annulaire, placé devant ce tube 8.

Le deuxième système de polarisation 14 peut être fixe relativement au miroir 6 et c'est ce dernier qui peut tourner relativement au premier système de polarisation 11.

Le système de repérage comporte par exemple une pièce annulaire 22 entourant le miroir 6 et sur laquelle les graduations 18 sont portées.

Le miroir 6 peut être solidaire d'un organe d'entraînement 23 qui sert également d'index en se déplaçant devant les graduations 18.

La figure 3 illustre la possibilité pour le deuxième système de polarisation 14 de présenter une direction de polarisation 15 fixe tandis que celle du premier système de polarisation 11 peut être modifiée.

Dans l'exemple de cette figure, le premier système de polarisation 11 présente une forme annulaire, pouvant être entraîné en rotation par un organe d'entraînement 25 relativement au miroir 6 et à un boîtier 27 maintenu par un socle 28.

Afin de repérer l'écart angulaire entre les directions de polarisation, le premier système de polarisation 11 peut par exemple tourner avec un cylindre (non apparent sur la figure) portant des graduations ou d'autres types d'inscriptions apparaissant à travers une fenêtre 30 réalisée dans le boîtier 27, selon l'orientation angulaire de l'organe de préhension 25.

Ainsi, l'utilisateur peut, en regardant la graduation apparaissant dans la fenêtre 30, connaître la position angulaire du premier système de polarisation 11 relativement au deuxième système de polarisation 14.

Des fibres optiques (non représentées) peuvent être utilisées pour véhiculer la lumière entre une lampe (non représentée) logée dans le socle 28 et le couvercle 27, les fibres optiques ayant par exemple leurs extrémités réparties tout autour du miroir 6 de manière à produire un éclairage uniforme.

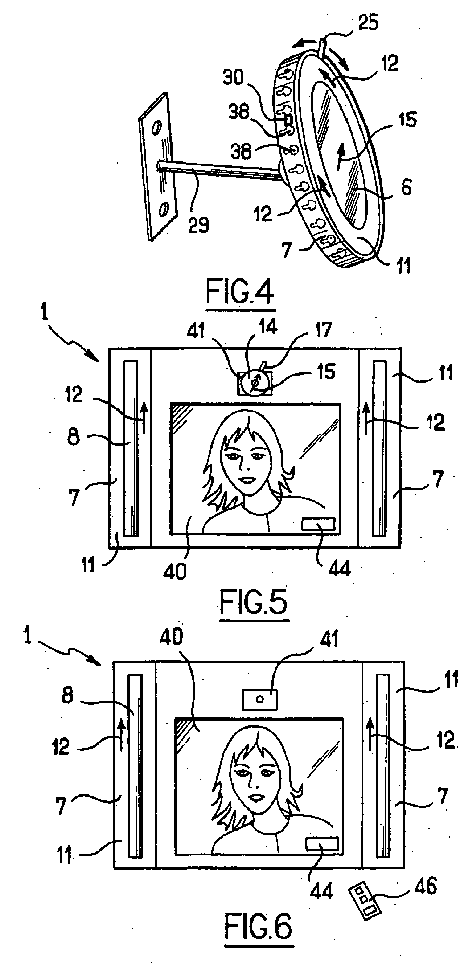

La variante de réalisation de la figure 4 diffère de l'exemple de réalisation de la figure 3 par le fait que le socle 28 est remplacé par une attache murale 29 et par le fait que la source 7 comporte une pluralité de diodes électroluminescentes 38, lesquelles sont par exemple réparties tout autour du miroir 6 derrière le premier système de polarisation 11.

Le système de polarisation 6 peut encore ne pas comporter de miroir, celui-ci étant remplacé par un système vidéo comportant, comme illustré sur les figures 5 et 6, un écran 40 et une caméra 41, l'image affichée sur l'écran 40 étant celle observée par la caméra 41.

Le dispositif 1 de la figure 5 comporte deux sources 7 disposées de part et d'autre de l'écran 40, et munies chacune du premier système de polarisation 11.

Le deuxième système de polarisation 14 est par exemple placé devant la caméra 41 de manière à ce que l'image apparaissant sur l'écran 40 dépende de l'écart angulaire entre les directions de polarisation 12 et 15.

Le deuxième système de polarisation 14 est par exemple muni de l'organe d'entraînement 17 comme dans l'exemple de la figure 1 pour permettre à l'utilisateur de modifier son orientation.

Un capteur de position est par exemple associé au deuxième système de polarisation 14 et une information représentative de l'écart angulaire entre les directions 12 et 15 peut être affichée sur l'écran 40, par exemple dans une zone 44 de celui-ci.

L'écran 40 peut être de tout type, par exemple à cristaux liquides, à plasma ou à tube cathodique, étant couleur ou monochrome.

Dans l'exemple de la figure 6, le deuxième système de polarisation 14 de la figure 5 a été remplacé par un dispositif électronique (non représenté) agissant directement sur la caméra 41.

L'utilisateur peut par exemple commander ce dispositif électronique au moyen d'une télécommande 46 ou avec tout autre type d'interface, par exemple un clavier, une souris, une manette, un écran tactile, la voix, entre autres.

Le dispositif 1 peut comporter, quelle que soit la forme sous laquelle il est réalisé, des moyens permettant d'enregistrer une image de la personne telle qu'elle peut se voir, pour un écart angulaire donné des première et deuxième directions de polarisation 12 et 15.

Dans l'exemple des figures 5 et 6, cette image peut être enregistrée par exemple grâce à la caméra 41, celle-ci pouvant être reliée à un système d'acquisition d'images tel que par exemple un micro-ordinateur ou tout autre dispositif électronique approprié.

Dans le cas des dispositifs des figures 1 à 4 ou de tout autre dispositif dont le système de visualisation comporte un miroir, une caméra ou un appareil photographique peut être utilisé, associé à un système de polarisation.

La lumière nécessaire à la prise de vue peut provenir de la ou des sources 7 ou le cas échéant d'un ou plusieurs flashs, lesquels peuvent être munis d'un système de polarisation disposé de telle sorte que l'écart angulaire entre les première et deuxième directions de polarisation soit reproduit avec le système de prise de vue.

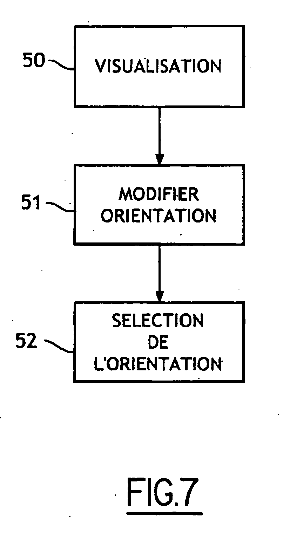

L'un des dispositifs qui vient d'être décrit peut par exemple être utilisé dans un procédé de sélection d'une apparence, tel qu'illustré à la figure 7.

Dans un tel procédé, on permet à une personne de se placer à une première étape 50 devant le système de visualisation.

On modifie ensuite, à l'étape 51, l'écart angulaire entre les première et deuxième directions de polarisation de manière à faire varier l'image que peut percevoir l'utilisateur, cette image étant par exemple réfléchie par le miroir 6 ou présentée à l'écran 40. C'est l'utilisateur lui-même qui peut modifier l'orientation ou en variante l'utilisateur peut donner, par exemple verbalement, des instructions à une autre personne chargée d'agir sur le dispositif sur ses indications.

L'orientation peut être modifiée jusqu'à ce que, à l'étape 52, l'utilisateur décide que l'apparence renvoyée lui paraît satisfaisante.

Les moyens de repérage peuvent être utilisés pour repérer l'orientation relative du premier système de polarisation par rapport au deuxième. Cette orientation peut servir par exemple de référence et l'on peut comparer les orientations sélectionnées à différents intervalles de temps.

On peut par exemple procéder à une première sélection de l'orientation en début de journée puis à une deuxième sélection de l'orientation plus tard dans la journée et mettre ainsi en évidence une évolution de la brillance de la peau au cours de la journée.

L'image correspondant à l'orientation sélectionnée par l'utilisateur peut être traitée, le cas échéant. Ce traitement peut par exemple comporter une comparaison avec l'image réelle, sans altération de la brillance par les systèmes de polarisation. On peut ainsi déterminer, par exemple, le besoin d'atténuation de la brillance et les zones à traiter.

La variation de l'angle peut par exemple renseigner sur la nature de la peau, les peaux très sèches étant peu sujettes à une augmentation de la brillance au cours de la journée.

Le dispositif 1 selon l'invention peut également être utile pour quantifier l'effet sur la brillance de la peau de l'application d'un produit.

On peut par exemple repérer l'écart angulaire entre les première et deuxième directions de polarisation avant l'application du produit puis après l'application.

L'effet matifiant d'un produit peut par exemple ainsi être caractérisé par l'angle dont on fait tourner l'un des systèmes de polarisation par rapport à l'autre entre les positions correspondant à avant et après l'application.

En effectuant une évaluation avant l'application d'un produit, en début de journée, puis une nouvelle évaluation en fin de journée, le produit ayant été appliqué le matin après la première évaluation, on peut mettre en évidence l'effet du produit sur la brillance de la peau en fin de journée, par exemple démontrer que le produit permet de conserver en fin de journée un niveau de brillance comparable à celui du début de la journée, avant application du produit.

Le dispositif selon l'invention peut faciliter la formulation d'un produit en permettant par exemple de déterminer la proportion des différents constituants de ce produit qui permet d'obtenir une évolution prédéfinie, par exemple sensiblement nulle, de la brillance entre le début et la fin de la journée, et ainsi fabriquer un produit adapté à un utilisateur ou à une population donnée.

Par exemple, selon l'orientation sélectionnée par une personne ou celle représentative d'une population, on pourra formuler un produit avec plus ou moins de charges matifiantes ou de composés conférant de la brillance.

Bien entendu, l'invention n'est pas limitée aux exemples qui viennent d'être décrits.

Dans tous les exemples qui précèdent, la source lumineuse peut être modifiée et comporter par exemple un tube fluorescent, une lampe à incandescence, des diodes électroluminescentes, avec des fibres optiques le cas échéant pour véhiculer la lumière.

Le premier système de polarisation peut encore être intégré à la source lumineuse ou celle-ci émettre une lumière polarisée.

La source lumineuse peut encore être la lumière du jour.

Le cas échéant, le dispositif peut comporter une source capable d'émettre plusieurs types de lumière, correspondant par exemple à des éclairages de type lumière du jour, à fluorescence ou à incandescence.

Le dispositif peut être intégré à une chaîne d'analyse plus complexe, renseignant par exemple l'utilisateur sur des défauts de sa peau ou la couleur de celle-ci.

L'invention n'est pas limitée à l'évaluation de la brillance de la peau et s'applique également à celle de la chevelure.

Dans toute la description, y compris les revendications, l'expression « comportant un » doit être comprise comme étant synonyme de « comportant au moins un », sauf si le contraire est spécifié.

## Revendications

1. Dispositif (1) comportant :
- un système de visualisation comportant l'un au moins
- d'un miroir (6) et
- d'un système vidéo comportant une caméra (41) et un écran (40) permettant de renvoyer vers un utilisateur placé devant la caméra une image acquise par la caméra,
- un premier système de polarisation (11) pour polariser la lumière émise par une source lumineuse dans une première direction (12),
- un deuxième système de polarisation (14) pour polariser la lumière reçue par le miroir ou la caméra dans une deuxième direction (15),
- des moyens pour faire varier l'écart angulaire entre les première et deuxième directions,
- un système de repérage (17 ; 30 ; 44) de l'écart angulaire permettant de repérer au moins trois écarts angulaires différents, **caractérisé en ce que** le système de repérage comporte des graduations.

2. Dispositif selon la revendication précédente, **caractérisé par le fait qu'**il comporte la source lumineuse (7).

3. Dispositif selon l'une des deux revendications précédentes, **caractérisé par le fait que** la source lumineuse (7) est solidaire du système de visualisation.

4. Dispositif selon l'une quelconque des revendications précédentes, **caractérisé par le fait qu'**il comporte deux sources lumineuses (7) disposées de part et d'autre du système de visualisation.

5. Dispositif selon l'une quelconque des revendications 1 à 3, **caractérisé par le fait que** la source lumineuse (7) entoure au moins partiellement le système de visualisation.

6. Dispositif selon l'une quelconque des revendications précédentes, **caractérisé par le fait que** la source lumineuse (7) émet une lumière diffuse.

7. Dispositif selon l'une quelconque des revendications précédentes, **caractérisé par le fait que** l'un du premier système de polarisation et du deuxième système de polarisation est rotatif.

8. Dispositif selon la revendication précédente, **caractérisé par le fait que** la rotation s'effectue par incréments.

9. Dispositif selon la revendication 7, **caractérisé par le fait que** la rotation s'effectue de manière continue.

10. Dispositif selon l'une quelconque des revendications 7 à 9, **caractérisé par le fait que** le système de polarisation qui est rotatif est configuré pour tourner entre deux positions extrêmes.

11. Dispositif selon l'une quelconque des revendications 7 à 9, **caractérisé par le fait que** le système de polarisation qui est rotatif est configuré pour pouvoir tourner sur un tour complet.

12. Dispositif selon l'une quelconque des revendications précédentes, **caractérisé par le fait que** le système de visualisation comporte un miroir (6).

13. Dispositif selon la revendication précédente, **caractérisé par le fait que** le miroir (6) est porté par un premier élément de structure (2) et **par le fait que** la source lumineuse (7) est portée par un deuxième élément de structure (3), mobile relativement au premier élément de structure.

14. Dispositif selon la revendication précédente, **caractérisé par le fait que** le deuxième élément de structure (3) est articulé sur le premier élément de structure.

15. Dispositif selon l'une des revendications 13 et 14, **caractérisé par le fait que** le premier élément de structure et le deuxième élément de structure sont configurés pour se rabattre l'un sur l'autre.

16. Dispositif selon l'une quelconque des revendications 12 à 15, **caractérisé par le fait que** le miroir est circulaire.

17. Dispositif selon l'une quelconque des revendications précédentes, **caractérisé par le fait que** le deuxième système de polarisation (14) est rotatif.

18. Dispositif selon la revendication 17, **caractérisé par le fait que** le miroir est non rotatif et **par le fait que** le deuxième système de polarisation (14) tourne relativement au miroir (6).

19. Dispositif selon la revendication 17, **caractérisé par le fait que** le miroir est rotatif et **par le fait que** le deuxième système de polarisation (14) tourne avec le miroir (6).

20. Dispositif selon l'une quelconque des revendications 1 à 16, **caractérisé par le fait que** le premier système de polarisation (11) est rotatif.

21. Dispositif selon l'une quelconque des revendications précédentes, **caractérisé par le fait que** le premier système de polarisation (11) est de forme annulaire.

22. Dispositif selon l'une quelconque des revendications 1 à 11, **caractérisé par le fait que** le système de visualisation comporte le système vidéo.

23. Dispositif selon l'une quelconque des revendications précédentes, **caractérisé par le fait que** le système de repérage comporte un index disposé sur le deuxième système de polarisation.

24. Dispositif selon l'une quelconque des revendications précédentes, **caractérisé par le fait que** le système de repérage comporte des moyens permettant de mémoriser une position.

25. Dispositif selon l'une quelconque des revendications précédentes, **caractérisé** le fait que le système de repérage comporte un capteur de position angulaire.

26. Dispositif selon l'une quelconque des revendications précédentes, **caractérisé par le fait qu'**il comporte des moyens d'affichage d'une information relative à l'écart angulaire entre les première et seconde directions de polarisation.

27. Dispositif selon l'une quelconque des revendications précédentes, **caractérisé** le fait qu'il comporte un système de prise de vue pour enregistrer une image de la personne correspondant à l'apparence sélectionnée, notamment un système de prise de vue comportant un premier système de polarisation sur un flash et un deuxième système de polarisation sur un objectif d'un appareil de prise de vue.

28. Procédé de détermination d'au moins un niveau de brillance du visage d'une personne sélectionné par celle-ci, comportant les étapes suivantes :
- permettre à la personne de visualiser son visage avec un dispositif comportant :
- un système de visualisation comportant l'un au moins d'un miroir et d'un système vidéo comportant une caméra devant laquelle la personne est placée et un écran permettant de renvoyer vers la personne l'image acquise par la caméra,
- un premier système de polarisation pour polariser la lumière émise par une source lumineuse dans une première direction, afin d'éclairer le visage,
- un deuxième système de polarisation pour polariser la lumière reçue par le système de visualisation, dans une deuxième direction,
- des moyens pour faire varier l'écart angulaire entre les première et deuxième directions et le niveau de brillance associé,
- permettre à la personne de modifier ou faire modifier l'écart angulaire entre les première et deuxième directions de polarisation, de manière à sélectionner au moins une apparence,
- déterminer l'écart angulaire entre les première et deuxième directions de polarisation, ledit écart étant représentatif du niveau de brillance sélectionné par la personne.

29. Procédé selon la revendication précédente, **caractérisé par le fait que** l'apparence sélectionnée correspond à l'apparence préférée par cette personne.

30. Procédé selon la revendication 28, **caractérisé par le fait que** la personne sélectionne une plage d'apparences acceptées par elle.

31. Procédé selon la revendication 28, **caractérisé par le fait que** la personne sélectionne une plage d'apparences non acceptées par elle.

32. Procédé selon l'une des revendications 28 à 31, précédentes, **caractérisé par le fait qu'**il est mis en oeuvre alors que le visage est non maquillé.

33. Procédé selon l'une des revendications 28 à 31, **caractérisé par le fait qu'**il est mis en oeuvre alors que le visage est maquillé.

34. Procédé selon l'une quelconque des revendications 28 à 33, **caractérisé par le fait que** l'on enregistre l'écart angulaire sélectionné ou une information représentative de celle-ci.

35. Procédé selon l'une quelconque des revendications 28 à 34, **caractérisé par le fait que** l'on délivre en fonction de l'écart angulaire une information concernant la peau et/ou les cheveux de la personne.

36. Procédé selon l'une quelconque des revendications 28 à 35 **caractérisé par le fait que** l'on délivre à la personne un produit ou un conseil d'achat d'un produit en fonction de l'écart angulaire déterminé.

37. Procédé selon la revendication 36, **caractérisé par le fait que** le produit est choisi parmi plusieurs produits existants.

38. Procédé selon la revendication 37, **caractérisé par le fait que** les produits existants sont respectivement associés à des valeurs ou plages de valeurs représentatives de différences entre des écarts angulaires sélectionnés et un angle de 45° entre les première et deuxième directions de polarisation.

39. Procédé selon la revendication 36, **caractérisé par le fait que** le produit est un produit personnalisé formulé *in situ*.

40. Procédé pour mettre en évidence une variation de l'apparence d'une portion du corps d'une personne, notamment son visage, en fonction du temps et/ou d'un traitement, comportant les étapes suivantes :
- permettre à la personne de visualiser cette portion de son corps avec un dispositif comportant :
- un système de visualisation comportant l'un au moins d'un miroir et d'un système vidéo comportant une caméra devant laquelle la personne est placée et un écran permettant de renvoyer vers cette personne une image acquise par la caméra,
- un premier système de polarisation (11) pour polariser la lumière émise par une source lumineuse dans une première direction (12),
- un deuxième système de polarisation (14) pour polariser la lumière reçue par le système de visualisation dans une deuxième direction (15),
- des moyens pour faire varier l'écart angulaire entre les première et deuxième directions,
- permettre à la personne de modifier l'écart angulaire entre les directions de polarisation de manière à sélectionner un premier écart angulaire correspondant à une apparence préférée dans un premier intervalle de temps,
- permettre à la personne de sélectionner un deuxième écart angulaire correspondant à une apparence préférée dans un deuxième intervalle de temps différent du premier, et
- comparer les premier et deuxième écarts angulaires sélectionnés ou une information représentative de ceux-ci.

41. Procédé selon la revendication précédente, **caractérisé par le fait qu'**on applique un produit sur la personne entre le premier et le deuxième intervalles de temps.

42. Procédé selon l'une des deux revendications précédentes, **caractérisé par le fait qu'**on délivre une information concernant un effet du produit en fonction de la comparaison.

43. Procédé selon l'une des revendications 40 à 42, **caractérisé par le fait que** le premier intervalle de temps est choisi le matin d'une journée donnée et le deuxième intervalle de temps le soir de cette journée.

44. Procédé de formulation d'un produit comportant l'étape consistant à formuler le produit en fonction d'un écart angulaire déterminé à la revendication 28.

45. Procédé selon la revendication précédente, **caractérisé par le fait que** l'écart angulaire sélectionné correspond à une apparence préférée de la personne.

46. Procédé selon l'une des deux revendications précédentes, **caractérisé par le fait qu'**on incorpore dans le produit une charge matifiante dans une proportion choisie en fonction de l'orientation sélectionnée.

47. Procédé selon l'une des trois revendications précédentes, **caractérisé par le fait qu'**on incorpore dans le produit une charge d'un composé conférant de la brillance dans une proportion choisie en fonction de l'orientation sélectionnée.

## Claims

1. A device (1) comprising:
· a display system comprising at least one of:
· a mirror (6); and
· a video system comprising a camera (41) and a screen (40) for returning to a user positioned in front of the camera, an image acquired by the camera;
· a first polarizing system (11) for polarizing in a first direction (12) the light emitted by a light source;
· a second polarizing system (14) for polarizing in a second direction (15) the light received by the mirror or by the camera;
· means for varying the angular offset between the first and second directions; and
· an angular-offset identifier system (12; 17; 30; 44) for identifying at least three different angular offsets,
**characterized by** the fact that the angular-offset identifier comprises graduations.

2. A device according to the preceding claim, **characterized by** the fact that it comprises the light source (7).

3. A device according to any one of the two preceding claims, **characterized by** the fact that the light source (7) is secured to the display system.

4. A device according to any preceding claim, **characterized by** the fact that it comprises two light sources (7) disposed on either side of the display system.

5. A device according to any one of claims 1 to 3, **characterized by** the fact that the light source (7) surrounds the display system, at least in part.

6. A device according to any preceding claim, **characterized by** the fact that the light source (7) emits diffuse light.

7. A device according to any preceding claim, **characterized by** the fact that one of the first and second polarizing systems is rotary.

8. A device according to the preceding claim, **characterized by** the fact that rotation is performed incrementally.

9. A device according to claim 7, **characterized by** the fact that rotation is performed continuously.

10. A device according to any one of claims 7 to 9, **characterized by** the fact that the rotary polarizing system is configured to turn between two extreme positions.

11. A device according to any one of claims 7 to 9, **characterized by** the fact that the rotary polarizing system is configured to be capable of turning through a complete turn.

12. A device according to any preceding claim, **characterized by** the fact that the display system comprises a mirror (6).

13. A device according to the preceding claim, **characterized by** the fact that the mirror (6) is carried by a first structure element (2), and by the fact that the light source (7) is carried by a second structure element (3) that is movable relative to the first structure element.

14. A device according to the preceding claim, **characterized by** the fact that the second structure element (3) is hinged onto the first structure element.

15. A device according to claim 13 or claim 14, **characterized by** the fact that the first structure element and the second structure element are configured to be folded one on the other.

16. A device according to any one of claims 12 to 15, **characterized by** the fact that the mirror is circular.

17. A device according to any preceding claim, **characterized by** the fact that the second polarizing system (14) is rotary.

18. A device according to claim 17, **characterized by** the fact that the mirror is not rotary, and by the fact that the second polarizing system (14) turns relative to the mirror (6).

19. A device according to claim 17, **characterized by** the fact that the mirror is rotary, and by the fact that the second polarizing system (14) turns with the mirror (6).

20. A device according to any one of claims 1 to 16, **characterized by** the fact that the first polarizing system (11) is rotary.

21. A device according to any preceding claim, **characterized by** the fact that the first polarizing system (11) is of annular shape.

22. A device according to any one of claims 1 to 11, **characterized by** the fact that the display system comprises a video system.

23. A device according to any preceding claim, **characterized by** the fact that the identifier system comprises an index disposed on the second polarizing system.

24. A device according to any preceding claim, **characterized by** the fact that the identifier system comprises memory means for memorizing a position.

25. A device according to any preceding claim, **characterized by** the fact that the identifier system comprises an angular-position sensor.

26. A device according to any preceding claim, **characterized by** the fact that it comprises display means for displaying information relating to the angular offset between the first and second polarizing directions.

27. A device according to any preceding claim, **characterized by** the fact that it comprises a camera for recording an image of the person corresponding to the selected appearance, in particular a camera comprising a first polarizing system on a flash gun and a second polarizing system on a camera lens.

28. A method of determining at least one level of shine on the face of a person, with said level of shine being selected by said person, said method comprising the following steps:
· enabling the face of the person to be displayed using a device comprising:
· a display system comprising at least one of a mirror and a video system comprising a camera in front of which the person is positioned, and a screen for returning to the person, the image acquired by the camera;
· a first polarizing system for polarizing in a first direction the light emitted by a light source, so as to illuminate the face;
· a second polarizing system for polarizing in a second direction the light received by the display system; and
· means for varying the angular offset between the first and second directions, and varying the associated level of shine;
· enabling the person to modify the angular offset between the first and second polarizing directions, or to cause said angular offset to be modified, so as to select at least one appearance; and
· determining the angular offset between the first and second polarizing directions, said offset being representative of the level of shine selected by the person.

29. A method according to the preceding claim, **characterized by** the fact that the selected appearance corresponds to the appearance that is preferred by the person.

30. A method according to claim 28, **characterized by** the fact that the person selects appearances in an acceptable range.

31. A method according to claim 28, **characterized by** the fact that the person selects appearances in an unacceptable range.

32. A method according to any one of claims 28 to 31, **characterized by** the fact that it is implemented when the face is not made up.

33. A method according to any one of claims 28 to 31, **characterized by** the fact that it is implemented when the face is made up.

34. A method according to any one of claims 28 to 33, **characterized by** the fact that the selected angular offset, or information that is representative of said angular offset, is recorded.

35. A method according to any one of claims 28 to 34, **characterized by** the fact that information concerning the skin and/or the hair of the person is supplied as a function of the angular offset.

36. A method according to any one of claims 28 to 35, **characterized by** the fact that a cosmetic, or advice about buying a cosmetic, is supplied to the person as a function of the determined angular offset.

37. A method according to claim 36, **characterized by** the fact that the cosmetic is selected from a plurality of existing cosmetics.

38. A method according to claim 37, **characterized by** the fact that the existing cosmetics are associated respectively with values or ranges of values that are representative of differences between selected angular offsets and an angle of 45° between the first and second polarizing directions.

39. A method according to claim 36, **characterized by** the fact that the cosmetic is a personalized cosmetic that is formulated *in situ.*

40. A method of demonstrating a variation in the appearance of a part of the body of a person, in particular the face, as a function of time and/or of a treatment, said method comprising the following steps:
· enabling the person to view said part of the body with a device comprising:
· a display system comprising at least one of a mirror and a video system comprising a camera in front of which the person is positioned, and a screen for returning to the person, an image acquired by the camera;
· a first polarizing system (11) for polarizing in a first direction (12) the light emitted by a light source;
· a second polarizing system (14) for polarizing in a second direction (15) the light received by the display system; and
means for varying the angular offset between the first and second directions;
· enabling the person to modify the angular offset between the polarizing directions so as to select a first angular offset corresponding to a preferred appearance in a first time interval;
· enabling the person to select a second angular offset corresponding to a preferred appearance in a second time interval that is different from the first; and
· comparing the selected first and second angular offsets, or information that is representative of said selected angular offsets.

41. A method according to the preceding claim, **characterized by** the fact that a cosmetic is applied to the person between the first and the second time intervals.

42. A method according to any one of the two preceding claims, **characterized by** the fact that information is supplied concerning an effect of the cosmetic as a function of the comparison.

43. A method according to any one of claims 40 to 42, **characterized by** the fact that the first time interval is selected to be in the morning of a given day, and the second time interval is selected to be in the evening of the same day.

44. A method of formulating a cosmetic, said method comprising the step consisting in formulating the cosmetic as a function of an angular offset determined in claim 28.

45. A method according to the preceding claim, **characterized by** the fact that the selected angular offset corresponds to an appearance that is preferred by the person.

46. A method according to any one of the two preceding claims, **characterized by** the fact that a shine-controlling filler is incorporated in the cosmetic in a proportion selected as a function of the selected orientation.

47. A method according to any one of the three preceding claims, **characterized by** the fact that a filler of a compound imparting shine is incorporated in the cosmetic in a proportion selected as a function of the selected orientation.

## Patentansprüche

1. Vorrichtung (1) umfassend:
- ein Visualisierungssystem, umfassend mindestens eines
- von einem Spiegel (6) und
- von einem Videosystem, das eine Kamera (41) und einen Bildschirm (40) umfasst, der es gestattet, zu einem vor der Kamera befindlichen Benutzer ein von der Kamera aufgenommenes Bild zurückzusenden,
- ein erstes Polarisierungssystem (11) zum Polarisieren des von einer Lichtquelle gesendeten Lichts in einer ersten Richtung (12),
- ein zweites Polarisierungssystem (14) zum Polarisieren des von dem Spiegel oder der Kamera empfangenen Lichts in einer zweiten Richtung (15),
- Mittel zum Ändern des Winkelabstands zwischen der ersten und der zweiten Richtung,
- ein System (17; 30; 44) zum Markieren des Winkelabstands, das die Markierung von mindestens drei verschiedenen Winkelabweichungen gestattet, **dadurch gekennzeichnet, dass** das Markierungssystem Gradteilungen umfasst.

2. Vorrichtung nach dem vorhergehenden Anspruch, **dadurch gekennzeichnet, dass** sie die Lichtquelle (7) umfasst.

3. Vorrichtung nach einem der beiden vorhergehenden Ansprüche, **dadurch gekennzeichnet, dass** die Lichtquelle (7) mit dem Visualisierungssystem fest verbunden ist.

4. Vorrichtung nach einem der vorhergehenden Ansprüche, **dadurch gekennzeichnet, dass** sie zwei Lichtquellen (7) umfasst, die zu beiden Seiten des Visualisierungssystems angeordnet sind.

5. Vorrichtung nach einem der Ansprüche 1 bis 3, **dadurch gekennzeichnet, dass** die Lichtquelle (7) das Visualisierungssystem mindestens teilweise umgibt.

6. Vorrichtung nach einem der vorhergehenden Ansprüche, **dadurch gekennzeichnet, dass** die Lichtquelle (7) ein diffuses Licht sendet.

7. Vorrichtung nach einem der vorhergehenden Ansprüche, **dadurch gekennzeichnet, dass** eines des ersten Polarisierungssystems und des zweiten Polarisierungssystems drehbar ist.

8. Vorrichtung nach dem vorhergehenden Anspruch, **dadurch gekennzeichnet, dass** die Drehung in Inkrementen stattfindet.

9. Vorrichtung nach Anspruch 7, **dadurch gekennzeichnet, dass** die Drehung kontinuierlich stattfindet.

10. Vorrichtung nach einem der vorhergehenden Ansprüche 7 bis 9, **dadurch gekennzeichnet, dass** das Polarisierungssystem, das drehbar ist, ausgebildet ist, um sich zwischen zwei Endstellungen zu drehen.

11. Vorrichtung nach einem der Ansprüche 7 bis 9, **dadurch gekennzeichnet, dass** das Polarisierungssystem, das drehbar ist, ausgebildet ist, um sich über eine vollständige Umdrehung drehen zu können.

12. Vorrichtung nach einem der vorhergehenden Ansprüche, **dadurch gekennzeichnet, dass** das Visualisierungssystem einen Spiegel (6) umfasst.

13. Vorrichtung nach dem vorhergehenden Anspruch, **dadurch gekennzeichnet, dass** der Spiegel (6) von einem ersten Strukturelement (2) getragen ist und dass die Lichtquelle (7) von einem zweiten Strukturelement (3) getragen ist, das bezüglich des ersten Strukturelements beweglich ist.

14. Vorrichtung nach dem vorhergehenden Anspruch, **dadurch gekennzeichnet, dass** das zweite Strukturelement (3) an dem ersten Strukturelement angelenkt ist.

15. Vorrichtung nach einem der Ansprüche 13 und 14, **dadurch gekennzeichnet, dass** das erste Strukturelement und das zweite Strukturelement ausgebildet sind, um zusammengeklappt zu werden.

16. Vorrichtung nach einem der Ansprüche 12 bis 15, **dadurch gekennzeichnet, dass** der Spiegel kreisförmig ist.

17. Vorrichtung nach einem der vorhergehenden Ansprüche, **dadurch gekennzeichnet, dass** das zweite Polarisierungssystem (14) drehbar ist.

18. Vorrichtung nach Anspruch 17, **dadurch gekennzeichnet, dass** der Spiegel nicht drehbar ist und dass das zweite Polarisierungssystem (14) sich bezüglich des Spiegels (6) dreht.

19. Vorrichtung nach Anspruch 17, **dadurch gekennzeichnet, dass** der Spiegel drehbar ist und dass das zweite Polarisierungssystem (14) sich mit dem Spiegel (6) dreht.

20. Vorrichtung nach einem der Ansprüche 1 bis 16, **dadurch gekennzeichnet, dass** das erste Polarisierungssystem (11) drehbar ist.

21. Vorrichtung nach einem der vorhergehenden Ansprüche, **dadurch gekennzeichnet, dass** das erste Polarisierungssystem (11) ringförmig ist.

22. Vorrichtung nach einem der Ansprüche 1 bis 11, **dadurch gekennzeichnet, dass** das Visualisierungssystem das Videosystem umfasst.

23. Vorrichtung nach einem der vorhergehenden Ansprüche, **dadurch gekennzeichnet, dass** das Markierungssystem einen Zeiger umfasst, der auf dem zweiten Polarisierungssystem angeordnet ist.

24. Vorrichtung nach einem der vorhergehenden Ansprüche, **dadurch gekennzeichnet, dass** das Markierungssystem Mittel umfasst, die die Speicherung einer Position gestatten.

25. Vorrichtung nach einem der vorhergehenden Ansprüche, **dadurch gekennzeichnet, dass** das Markierungssystem einen Winkelstellungsfühler umfasst.

26. Vorrichtung nach einem der vorhergehenden Ansprüche, **dadurch gekennzeichnet, dass** sie Mittel zum Anzeigen einer Information über die Winkelabweichung zwischen der ersten und der zweiten Polarisierungsrichtung umfasst.

27. Vorrichtung nach einem der vorhergehenden Ansprüche, **dadurch gekennzeichnet, dass** sie ein Bildaufnahmesystem zum Aufzeichnen eines Bilds der Person umfasst, das dem ausgewählten Aussehen entspricht, insbesondere ein Bildaufnahmesystem, das ein erstes Polarisierungssystem auf einem Blitzlicht und ein zweites Polarisierungssystem auf einem Objektiv eines Bildaufnahmegeräts umfasst.

28. Verfahren zur Bestimmung mindestens einer Glanzstufe des Gesichts einer Person, die von dieser ausgewählt wird, umfassend die folgenden Schritte:
- der Person gestatten, ihr Gesicht mit einer Vorrichtung zu visualisieren, umfassend:
- ein Visualisierungssystem, umfassend mindestens eines von einem Spiegel und einem Videosystem, das eine Kamera, vor der sich die Person befindet, und einen Bildschirm umfasst, der es gestattet, das von der Kamera aufgenommene Bild zu der Person zurückzusenden,
- ein erstes Polarisierungssystem zum Polarisieren des von einer Lichtquelle gesendeten Lichts in einer ersten Richtung, um das Gesicht zu beleuchten,
- ein zweites Polarisierungssystem zum Polarisieren des von dem Visualisierungssystem empfangenen Lichts in einer zweiten Richtung,
- Mittel zum Ändern des Winkelabstands zwischen der ersten und der zweiten Richtung und der zugeordneten Glanzstufe,
- der Person gestatten, die Winkelabweichung zwischen der ersten und der zweiten Polarisierung so zu ändern oder ändern zu lassen, dass mindestens ein Aussehen ausgewählt wird,
- die Winkelabweichung zwischen der ersten und der zweiten Polarisierungsrichtung bestimmen, wobei diese Abweichung für die von der Person ausgewählten Glanzstufe repräsentativ ist.

29. Verfahren nach dem vorhergehenden Anspruch, **dadurch gekennzeichnet, dass** das ausgewählte Aussehen dem von dieser Person bevorzugten Aussehen entspricht.

30. Verfahren nach Anspruch 28, **dadurch gekennzeichnet, dass** die Person einen Bereich von Aussehen, die von ihr akzeptiert werden, wählt.

31. Verfahren nach Anspruch 28, **dadurch gekennzeichnet, dass** die Person einen Bereich von Aussehen wählt, die von ihr nicht akzeptiert werden.

32. Verfahren nach einem der Ansprüche 28 bis 31, **dadurch gekennzeichnet, dass** es durchgeführt wird, während das Gesicht nicht geschminkt ist.

33. Verfahren nach einem der Ansprüche 28 bis 31, **dadurch gekennzeichnet, dass** es durchgeführt wird, während das Gesicht geschminkt ist.

34. Verfahren nach einem der Ansprüche 28 bis 33, **dadurch gekennzeichnet, dass** man die gewählte Winkelabweichung oder eine für diese repräsentative Information aufzeichnet.

35. Verfahren nach einem der Ansprüche 28 bis 34, **dadurch gekennzeichnet, dass** man in Abhängigkeit von der Winkelabweichung eine die Haut und/oder die Haare der Person betreffende Information liefert.

36. Verfahren nach einem der Ansprüche 28 bis 35, **dadurch gekennzeichnet, dass** man der Person ein Produkt oder eine Kaufempfehlung für ein Produkt in Abhängigkeit von der bestimmten Winkelabweichung liefert.

37. Verfahren nach Anspruch 36, **dadurch gekennzeichnet, dass** das Produkt aus mehreren bestehenden Produkten ausgewählt ist und.

38. Verfahren nach Anspruch 37, **dadurch gekennzeichnet, dass** die bestehenden Produkte jeweils Werten oder Wertebereichen zugeordnet sind, die für Differenzen zwischen ausgewählten Winkelabweichungen und einem Winkel von 45° zwischen der ersten und zweiten Polarisierungsrichtung repräsentativ sind.

39. Verfahren nach Anspruch 36, **dadurch gekennzeichnet, dass** das Produkt ein in situ formuliertes personalisiertes Produkt ist.

40. Verfahren zur Erfassung einer Änderung des Aussehens eines Körperteils einer Person, insbesondere ihres Gesichts, in Abhängigkeit von der Zeit und/oder einer Behandlung, umfassend die folgenden Schritte:
- der Person gestatten, diesen Teil ihres Körpers mit einer Vorrichtung zu visualisieren, die umfasst:
- ein Visualisierungssystem, das mindestens eines von einem Spiegel und einem Videosystem umfasst, das eine Kamera, vor der sich die Person befindet, und einen Bildschirm umfasst, der es gestattet, zu dieser Person ein von der Kamera aufgenommenes Bild zurückzusenden,
- ein erstes Polarisierungssystem (11) zum Polarisieren des von einer Lichtquelle gesendeten Lichts in einer ersten Richtung (12),
- ein zweites Polarisierungssystem (14) zum Polarisieren des von dem Visualisierungssystem empfangenen Lichts in einer zweiten Richtung (15),
- Mitteln zum Ändern der Winkelabweichung zwischen der ersten und der zweiten Richtung,
- der Person gestatten, die Winkelabweichung zwischen den Polarisierungsrichtungen so zu ändern, dass eine erste Winkelabweichung ausgewählt wird, die einem bevorzugten Aussehen in einem ersten Zeitintervall entspricht,
- der Person gestatten, eine zweite Winkelabweichung auszuwählen, die einem bevorzugten Aussehen in einem zweiten, von dem ersten verschiedenen Zeitintervall entspricht, und
- die erste und die zweite ausgewählte Winkelabweichung oder eine für diese repräsentative Information vergleichen.

41. Verfahren nach dem vorhergehenden Anspruch, **dadurch gekennzeichnet, dass** man zwischen dem ersten und dem zweiten Zeitintervall auf die Person ein Produkt aufträgt.

42. Verfahren nach einem der vorhergehenden Ansprüche, **dadurch gekennzeichnet, dass** man eine Information liefert, die eine Wirkung des Produkts in Abhängigkeit von dem Vergleich betrifft.

43. Verfahren nach einem der Ansprüche 40 bis 42, **dadurch gekennzeichnet, dass** das erste Zeitintervall am Morgen eines bestimmten Tags und das zweite Zeitintervall am Abend dieses Tags gewählt wird.

44. Verfahren zur Formulierung eines Produkts, umfassend den Schritt, der darin besteht, dass das Produkt in Abhängigkeit von einer in Anspruch 28 bestimmten Winkelabweichung formuliert wird.

45. Verfahren nach dem vorhergehenden Anspruch, **dadurch gekennzeichnet, dass** die ausgewählte Winkelabweichung einem bevorzugten Aussehen der Person entspricht.

46. Verfahren nach einem der beiden vorhergehenden Ansprüche, **dadurch gekennzeichnet, dass** man in das Produkt eine Mattierungscharge in einem Anteil einbringt, der in Abhängigkeit von der ausgewählten Ausrichtung gewählt ist.

47. Verfahren nach einem der drei vorhergehenden Ansprüche, **dadurch gekennzeichnet, dass** man in das Produkt eine Charge einer Verbindung einbringt, die in einem in Abhängigkeit von der ausgewählten Ausrichtung gewählten Anteil Glanz verleiht.
